# EUROPÄISCHE PATENTANMELDUNG

(11) **EP 2 604 305 A1**
(43) Veröffentlichungstag der Anmeldung: **19.06.2013**
(21) Anmeldenummer: 12190721.6
(22) Anmeldetag: 31.10.2012
(51) Int. Cl.: A61M 25/00, A61B 18/14, A61M 25/01

(54) **Katheter und Verfahren zur Herstellung desselben**

(30) Priorität: 16.12.2011 US 201161576364 P
(71) Anmelder: VascoMed GmbH, 79589 Binzen (DE)
(72) Erfinder: Kaufmann, Ralf, 79540 Lörrach (DE); Schreiber, Siegfried, 79639 Grenzach-Wylen (DE); Rath-Prazak, Paul, 79206 Breisach (DE)
(74) Vertreter: Lindner-Vogt, Karin L.

(57) **Zusammenfassung**

Die Anmeldung betrifft einen Katheter mit einem Steuerungsseilanker (3), welcher vorzugsweise am distalen Ende des Katheters angeordnet ist, und mit einem Steuerungsseil (2). Um eine zuverlässige Verbindung zwischen Steuerungsseilanker (3) und Steuerungsseil (2) zu erhalten, die auch für die Anwendung im MRT geeignet ist, wird zwischen dem Steuerungsseilanker (3) und dem Steuerungsseil ein im Wesentlichen hantelförmiges Element (1) angeordnet, welches den Steuerungsseilanker (3) mit dem Steuerungsseil (2) verbindet.

## Beschreibung

Die vorliegende Erfindung betrifft eine Schutzhülse für einen Katheter mit einem Steuerungsseilanker, welcher vorzugsweise an distalen Enden des Katheters angeordnet ist, und einem Steuerungsseil sowie ein Verfahren zur Herstellung eines solchen Katheters.

Als Katheter werden im Allgemeinen Röhrchen oder Schläuche unterschiedlichen Durchmessers aus verschiedenen Materialien bezeichnet, mit denen Hohlorgane wie Harnblase, Magen, Darm, Blutgefäße oder das Herz sondiert, entleert, gefüllt oder gespült werden können. Eine spezielle Form von intravaskulär eingesetzten Kathetern, vor allem für die Anwendung im Herz-Thoraxbereich, sind Katheter mit einer Elektrode, welche in Hauptvenen oder -arterien, beispielsweise in die Vena Femoralis, eingeführt und von dort aus an unterschiedliche Stellen im Herzen bzw. in den Herzkranzgefäßen vorgeschoben werden. Diese Katheter werden dafür verwendet, die elektrische Aktivität des Herzens darzustellen oder zu stimulieren oder Bereiche mit abnormaler elektrischer Aktivität abzutragen. Die zuletzt genannte Ablationstherapie dient z.B. als Therapie gegen Herzrhythmusstörungen.

Zur Handhabung eines solchen Katheters wird der am distalen Ende des Katheters vorgesehene metallische Steuerungsseilanker, der beispielsweise als Elektrode ausgebildet ist, mit einem Steuerungsseil, das meist als Zugdraht ausgebildet ist, verbunden. Dieses Steuerungsseil wird von einem Kolben, der am proximalen Ende des Katheters angeordnet ist, bewegt und erstreckt sich vom proximalen Ende des Katheters bis zum distalen Ende. Eine Bewegung des Kolbens in Längsrichtung in Bezug auf die Kolbenkammer resultiert in einer Krümmung des Katheters im mittleren Bereich.

Bereits seit einiger Zeit bestehen Bemühungen, das Verfahren der Magnetresonanztomographie (MRT) nicht nur für die Bilddiagnostik, sondern auch zur Positionierungs- und Erfolgskontrolle simultan geführter Kathetereingriffe, beispielsweise bei therapeutischen Eingriffen gegen Herzrhythmusstörungen, einzusetzen. Dies erfordert Katheterkonstruktionen, die amagnetisch sind und die den Belastungen starker elektromagnetischer Wechselfelder standhalten. In derartigen Wechselfeldern dürfen sich die Katheterelemente weder verschieben oder erwärmen noch in mechanische Schwingungen geraten. Darüber hinaus sollen keine katheterbedingten Bildartefakte auftreten.

Eine Möglichkeit, ein intravaskulär eingesetztes Kathetersystem gegen die Einwirkungen eines sehr starken elektromagentischen Wechselfeldes zu schützen, ist, nichtmetallische Werkstoffe zu verwenden.

Bei den Bemühungen, metallische Elemente durch nichtmetallische Elemente zu ersetzen, wurde beobachtet, dass bei manuell steuerbaren Kathetern sich insbesondere das metallische Steuerungsseil, das im elektrischen Wechselfeld wie eine Antenne wirkt, an den Enden erhitzt und daher für eine Anwendung im MRT nicht geeignet ist. Wird das Steuerungsseil aus nichtmetallischen Materialien gefertigt, so ist für die sichere Funktion von elektrophysiologischen Kathetern besonders die Gestaltung einer zuverlässigen, dynamisch hoch belastbaren Verbindungstechnik zu entsprechenden Ankern, z.B. zu der vorzugsweise am distalen Ende des Katheters angeordneten Elektrode, kritisch.

Aus der Druckschrift EP 2 275 163 A2 ist ein Katheter mit einer Elektrode bekannt. In dem Dokument werden mehrere Möglichkeiten erläutert, wie eine Kopfelektrode mit einem Zugseil aus einem Material u. a. aus einem High Molecular Density Polyethylen verbunden werden kann. Eine in dem Dokument erläuterte und gezeigte Lösung verwendet einen quer zur Längsrichtung von der Kopfelektrode abragenden Dorn, um den der Zugdraht gewickelt und an dem der Zugdraht befestigt ist. Die bekannte Lösung ist jedoch sehr raumgreifend, da der Dorn senkrecht zur Katheterlängsachse abragt. Hierdurch wird der Platz für elektrische und hydraulische Leitungen im Katheterschaft eingeschränkt. Ferner biegt die durch das Zugseil aufgebrachte Zugbelastung den Dorn. Gegen eine derartige Biegebelastung sind Metalle wenig beständig, sodass die bekannte Verbindung als unsicher angesehen werden muss.

Die Aufgabe der vorliegenden Erfindung besteht demnach darin, einen Katheter zu schaffen, der im MRT eingesetzt werden kann und gleichzeitig zuverlässig sowie dynamisch hochbelastbar ist. Die Aufgabe der Erfindung besteht ferner darin, ein Verfahren zur Herstellung eines derartigen Katheters anzugeben, durch das ein solcher Katheter einfach und kostengünstig herstellbar ist.

Die obige Aufgabe wird durch den in Anspruch 1 angegebenen Gegenstand gelöst. Insbesondere weist der erfindungsgemäße Katheter ein zwischen dem Steuerungsseilanker und dem Steuerungsseil angeordnetes, im Wesentlichen hantelförmiges Element auf, welches die Elektrode mit dem Steuerungsseil verbindet.

Das vorzugsweise aus einem metallischen Material bestehende hantelförmige Element bildet bei dem vorliegenden erfindungsgemäßen Katheter ein Bauteil, das den metallischen Steuerungsseilanker mit dem nichtmetallischen, vorzugsweise aus einem hochfesten und dehnungsarmen Polymer bestehenden Steuerungsseil auf einfache Weise verbindet. Hierdurch wird eine statisch und dynamisch hoch belastbare Verbindung z.B. zwischen der als Zuganker wirkenden metallischen Elektrode, die insbesondere als Kopfelektrode ausgeführt ist, und dem Steuerungsseil hergestellt, so dass der elektrophysiologische Katheter gesteuert bzw. gebogen werden kann. Alternativ oder zusätzlich kann das hantelförmige Element Glas, Keramik und/oder einen Kunststoff, beispielsweise PEEK (Polyetheretherketon), enthalten.

Verglichen mit dem Steuerungsseil (z.B. in Form eines Zugdrahts) ist das hantelförmige Element kurz, so dass dieses, auch wenn es aus einem metallischen Material besteht, in den im MRT vorhandenen elektromagnetischen Wechselfeldern nicht als Antenne wirkt und keine Bildartefakte entstehen.

Das hantelförmige Element ist an dem einen proximalen Ende so gestaltet, dass das vorzugsweise als Zugseil ausgebildete Steuerungsseil mit einer Knotentechnik fest und eng anliegend angeschlagen werden kann. Das andere distale Ende des hantelförmigen Elements kann beispielsweise durch Löten, Schweißen, Einpressen, Einkleben oder Verschrauben mit dem Steuerungsseilanker, z.B. der Elektrode, verbunden werden. Hierdurch wird eine zuverlässige und dynamisch belastbare Verbindung zwischen Elektrode und Steuerungsseil hergestellt.

Weiterhin ist das hantelförmige Element so gestaltet, dass das Verbindungselement lediglich einen kleinen Teil des Katheterquerschnitts beansprucht. Auf diese Weise bleibt viel Raum für andere Funktionselemente, wie elektrische und/oder hydraulische Leitungen, und andere Katheterlumina frei. Insbesondere gilt dies bei einer Anordnung des hantelförmigen Elements in der Weise, dass seine Längsachse parallel oder fast parallel, d.h. unter kleinem Winkel, zur Längsachse des Katheters bzw. des Steuerungsseils verläuft.

Das hantelförmige Element weist vorzugsweise einen ersten Endabschnitt und einen zweiten Endabschnitt sowie einen dazwischen liegenden Mittelabschnitt auf, wobei vorzugsweise der erste Endabschnitt mit der Elektrode verbunden ist und an dem zweiten Endabschnitt das Steuerungsseil angeschlagen ist. Durch diese Gestaltung des hantelförmigen Elements lassen sich belastbare Verbindungen zur Elektrode einerseits und zum Steuerungsseil andererseits sicher und einfach herstellen.

Wie bereits oben ausgeführt wurde, enthält das Steuerungsseil ein Polymer, vorzugsweise ein hochkristallines, hochverstrecktes Ultra Hochmolekulares Polyethylen (UHMPE), und die Elektrode und/oder das hantelförmige Element ein metallisches Material, wobei das hantelförmige Element vorzugsweise nicht ferromagnetisch ausgebildet ist. Das hantelförmige Element ist z.B. aus einer Nickel-Titan-Legierung (NiTi-Legierung) gefertigt. Dies ist besonders vorteilhaft, da NiTi antimagnetisch ist und eine hohe Zugfestigkeit aufweist. Ein weiterer Vorteil der Verwendung von NiTi besteht darin, dass dieses korrosionsfest und kostengünstig ist. Ein solches hantelförmiges Element lässt sich durch Plasmastrahlschweißen oder Plasmastrahlschmelzen an den Enden eines entsprechenden Drahtabschnitts herstellen.

Der erste Endabschnitt und der zweite Endabschnitt des hantelförmigen Elements weisen gegenüber dem dazwischen liegenden Mittelabschnitt eine größere Dicke in eine Richtung quer zur Längsachse des hantelförmigen Elements (d.h. in radialer Richtung) auf, vorzugsweise einen maximalen Durchmesser in radialer Richtung des hantelförmigen Elements, der mindestens das Doppelte des Durchmessers des Mittelabschnitts in radialer Richtung beträgt. Hierdurch wird einerseits eine gute Möglichkeit geschaffen, das nichtmetallische Steuerungsseil mit dem Steuerungsseilanker sicher und dynamisch hochbelastbar, beispielsweise mittels eines Palsteks oder eines Bulin 1.5 als Schlaufe und einem ein- bis mehrfach gerollten Trompetenknoten an dem Mittelabschnitt anzuschlagen, und andererseits das hantelförmige Element mit einem großen Querschnitt mit dem Steuerungsseilanker sicher zu verbinden. Der Vorteil einer Verwendung eines Trompetenknotens bzw. Palsteks besteht darin, dass sich der Trompetenknoten bzw. der Palstek beim Auftreten von Zugkräften entlang des Steuerungsseils zusammenzieht.

Im Bereich der Verbindung zwischen dem ersten Endabschnitt und dem Steuerungsseil kann in dem ersten Endabschnitt und/oder dem Mittelabschnitt, vorzugsweise an dessen proximalen Ende, mindestens eine, besonders bevorzugt parallel zur Längsachse des Katheters verlaufende Nut in dem hantelförmigen Element vorgesehen sein, welche das Steuerungsseil an dem hantelförmigen Element entlang führt.

Vorzugsweise weist der Mittelabschnitt des hantelförmigen Elements einen Durchmesser von weniger als 1 mm und eine Länge von mindestens 3 mm, vorzugsweise eine Länge von mindestens 5 mm, besonders bevorzugt eine Länge von maximal 10 mm, auf. Aufgrund der gewählten Grenzen für die Abmessungen des hantelförmigen Elements bewirkt dieses einerseits eine sichere Verbindung zwischen Steuerungsseil und Elektrode als auch eine geringe Wechselwirkung mit den elektromagnetischen Wechselfeldern des MRT.

Die Verwendung eines Polymers, insbesondere eines hochkristallinen hoch verstreckten UHMPEs, für das Steuerungsseil ist vorteilhaft, da ein solches im elektromagnetischen Wechselfeld nicht störend ist und keine Antennenwirkung entfaltet. Ferner ist von Vorteil, dass die Biegeradien von Polymeren im Verhältnis zum Durchmesser viel kleiner sind. Weiterhin sind Polymere knicksicherer als metallisches Material, so dass kleinere Deflektionsradien des Katheters verwirklicht werden können. Hierdurch erhält der erfindungsgemäße Katheter eine höhere Biegeflexibilität.

In einer Weiterbildung der Erfindung ist der erste Endabschnitt des hantelförmigen Elements formschlüssig mit dem Steuerungsseilanker verbunden und z.B. in einer entsprechenden Ausnehmung des Steuerungsseilankers beispielsweise eingelötet, eingeschweißt oder verschraubt.

Der erste Endabschnitt und/oder der zweite Endabschnitt des hantelförmigen Elements sind beispielsweise als Kugel, Kugelabschnitt, Kegel, Kegelabschnitt oder Zylinder ausgebildet. Durch diese Ausbildung der Endabschnitte des hantelförmigen Elements ist es besonders einfach, den jeweiligen Abschnitt entweder in eine entsprechende Ausnehmung des Steuerungsseilankers anzuordnen oder mit dem Steuerungsseil durch Anschlagen eines Knotens oder einer Schlaufe zu verbinden. Der dem Steuerungsseil zugewandte Endabschnitt des hantelförmigen Elements bildet dabei den Anschlag für den Knoten oder die Schlaufe, über den der Knoten oder die Schlaufe aufgrund des großen Durchmessers nicht hinübergleiten kann. Entsprechend ist das Steuerungsseil an seinem distalen Ende vorzugsweise als Schlaufe ausgebildet, die um den zweiten Endabschnitt des hantelförmigen Elements geschlungen ist. Diese Verbindung zwischen Steuerungsseil und Steuerungsseilanker funktioniert ohne Verwendung von Klebstoffen, die u. a. die Eigenschaften eines polymeren Steuerungsseils negativ beeinflussen, z.B. mit diesem reagieren könnten.

Wie oben bereits ausgeführt wurde, verläuft die Längsachse des hantelförmigen Elements vorzugsweise parallel zur Katheterlängsachse, so dass die von dem Steuerungsseil aufgebrachten Kräfte keine Biegung des hantelförmigen Elements verursachen sondern lediglich eine Zugbelastung darstellen. Dies ist von Vorteil, da Metalle in der Regel verglichen mit einer Biegebelastung eine höhere Zugbelastung tolerieren. Mit der erfindungsgemäßen Anordnung sind daher hohe statische und zyklische Zuglasten bis zur Reißfestigkeit des Steuerungsseils in die Kopfelektrode bzw. den Steuerungsseilanker übertragbar. Ferner ist die Anordnung des hantelförmigen Elements auf diese Weise sehr platzsparend und stabil, da die Hantel genügend Freiraum für andere, bis zur Elektrode reichenden Signal- und Medienleitungen lässt.

Die obige Aufgabe wird zudem von einem Verfahren zur Herstellung eines Katheters mit einem Steuerungsseilanker, welcher vorzugsweise am distalen Ende des Katheters angeordnet ist und vorzugsweise eine Elektrode ausbildet, und mit einem Steuerungsseil mit den in Anspruch 10 angegebenen Schritten gelöst. Insbesondere weist das erfindungsgemäße Verfahren die folgenden Schritte auf:
a) Herstellen des Steuerungsseilankers und des Steuerungsseils,
b) Herstellen eines hantelförmigen Elements mit einem ersten Endabschnitt und einem zweiten Endabschnitt sowie einem dazwischen liegenden Mittelabschnitt,
c) Verbinden des Steuerungsseilankers mit dem ersten Endabschnitt des hantelförmigen Elements, vorzugweise über eine formschlüssige Verbindung, und
d) Verbinden des Steuerungsseils mit dem zweiten Endabschnitt des hantelförmigen Elements, vorzugsweise durch Anschlagen des Steuerungsseils an dem zweiten Endabschnitt.

Das obige erfindungsgemäße Verfahren ist ein einfaches Herstellungsverfahren, das zudem kostengünstig und reproduzierbar durchführbar ist. Vorzugsweise wird das Steuerungsseil mittels einer mehrfach angeschlungenen Schlaufe an dem zweiten Endabschnitt des hantelförmigen Elements befestigt.

Das Steuerungsseil kann außerdem in einem bevorzugten Ausführungsbeispiel zusätzlich mindestens eine vorzugsweise in Längsrichtung des Katheters verlaufende Nut zur Führung des Steuerungsseils aufweisen.

Besonders einfach lässt sich das erfindungsgemäße Herstellungsverfahren dadurch gestalten, dass das hantelförmige Element aus einem Draht, vorzugsweise aus einem Draht einer NiTi-Legierung, gefertigt wird, wobei der erste Endabschnitt und/oder der zweite Endabschnitt vorzugsweise mittels Plasmastrahlschweißen erzeugt werden. So wird auf einfache Weise eine Verdickung des zunächst im Mittelabschnitt vorhandenen Drahtes an dessen Ende erzeugt.

Weitere Ziele, Merkmale, Vorteile und Anwendungsmöglichkeiten der Erfindung ergeben sich aus der nachfolgenden Beschreibung eines Ausführungsbeispiels eines erfindungsgemäßen Katheters anhand der Figuren. Dabei bilden alle beschriebenen und/oder bildlich dargestellten Merkmale für sich oder in beliebiger Kombination den Gegenstand der vorliegenden Erfindung, unabhängig von ihrer Zusammenfassung in den einzelnen Ansprüchen oder deren Rückbeziehung.

Es zeigen schematisch:
- Fig. 1: eine Ansicht der Verbindung zwischen einer Kopfelektrode und einem Zugseil des in Figur 2 dargestellten Katheters in einer perspektivischen Ansicht von der Seite und
- Fig. 2: ein Ausführungsbeispiel eines erfindungsgemäßen Katheters in einer Ansicht von der Seite.

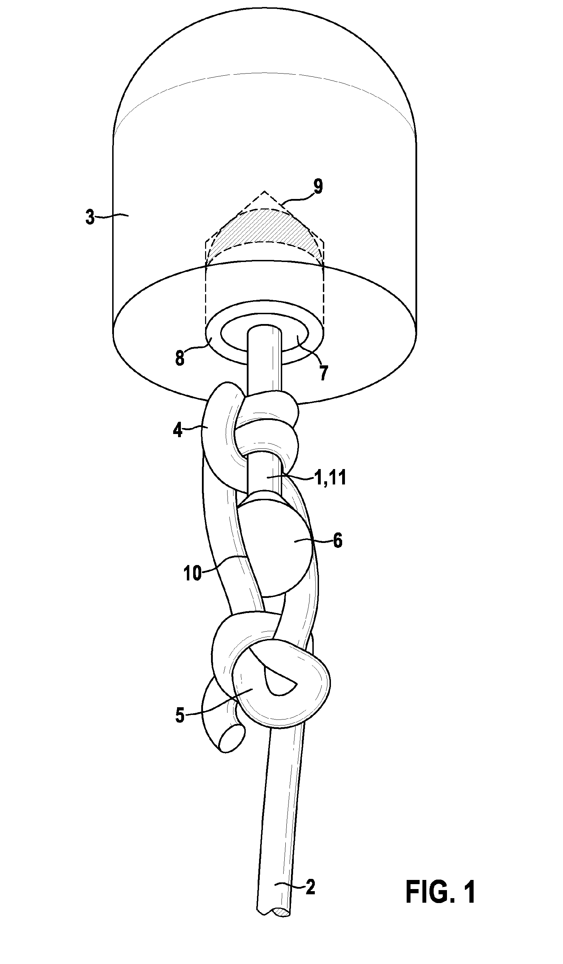

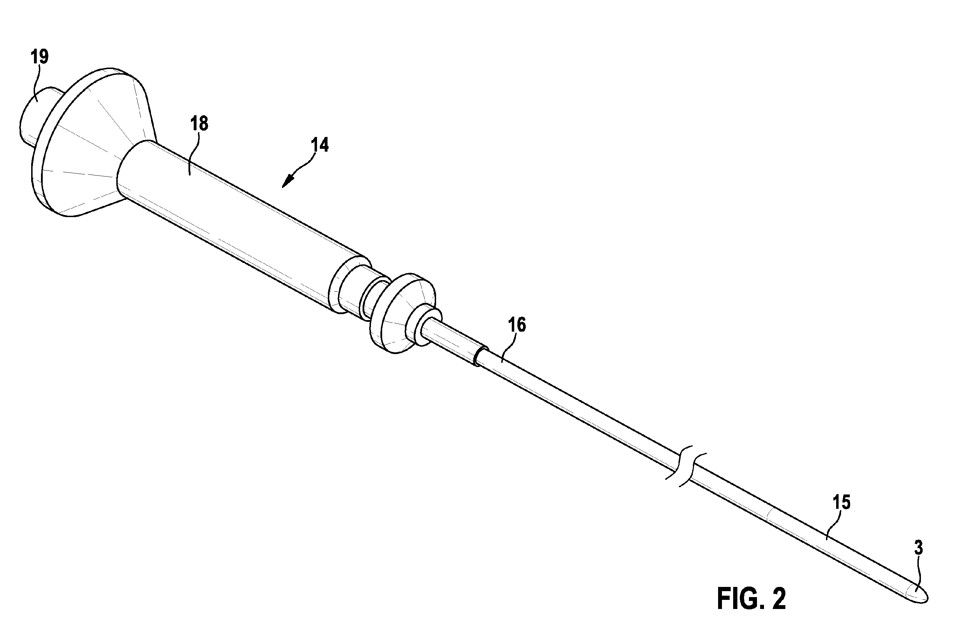

Figur 1 veranschaulicht die Verbindung zwischen einer im Wesentlichen zylinderförmigen Kopfelektrode 3 und einem Zugseil 2, wie sie erfindungsgemäß in dem in Figur 2 dargestellten Katheter vorliegt.

Nach der Erfindung ist ein hantelförmiges Element 1 mit einem ersten Endabschnitt 7, der bei dem gezeigten Ausführungsbeispiel in Form einer Kugel ausgestaltet ist, und einem zweiten Endabschnitt 6, welcher ebenfalls die Form einer Kugel hat, zwischen der Kopfelektrode 3 und dem Zugseil 2 vorgesehen. Zwischen dem ersten Endabschnitt 7 und dem zweiten Endabschnitt 6 des hantelförmigen Elements 1 ist der Mittelabschnitt 11, der drahtförmig ist, angeordnet. Der erste Endabschnitt 7 ist in eine im Wesentlichen zylinderförmige proximale Sacklochausnehmung 9 der Kopfelektrode 3, welche vorzugsweise in die Kopfelektrode 3 eingebohrt wurde, mit Hilfe von Lot 8 eingelötet.

Der Mittelabschnitt 11 des hantelförmigen Elements 1 hat in dem gezeigten Ausführungsbeispiel eine Runddraht-Form mit einem Durchmesser im Millimeterbereich, beispielsweise mit einem Durchmesser von 0,7 mm, und einer Länge von wenigen Millimetern, beispielsweise eine Länge im Bereich von 5 mm bis 8 mm.

Die an beiden Enden des Mittelabschnitts 11 angeordneten Kugeln 6, 7 weisen einen etwa 2- bis 3-fach größeren Durchmesser als der Mittelabschnitt 11 auf, wobei der Durchmesser jeweils in einer Richtung quer zur Katheterlängsachse (radiale Richtung) gemeint ist.

Der erste Endabschnitt 7 des hantelförmigen Elements 1 ist formschlüssig in einer zylindrischen Sacklochausnehmung 9 der Kopfelektrode 3 verankert. Der zweite Endabschnitt 6 dient als axialer Anschlag für eine mehrfach angeschlungene Schlaufe 4 des Zugseils 2. Das Zugseil 2 ist mit einer Schlaufe um den zweiten Endabschnitt 6 gelegt und an diesen mehrfach angeschlungen. Der zweite Endabschnitt 6 bildet aufgrund seiner Verdickung einen axialen Anschlag für die Schlaufe 4. Weiter bevorzugt kann der zweite Endabschnitt 6 mindestens eine, vorzugsweise zwei Nuten 10 aufweisen, in denen jeweils ein Steuerungsseil 2 angeordnet und zurückgeführt wird. Die Rückführung des Zugseils endet in einem Palstek 5, der sich bei höherer Zugbelastung an dem Zugseil 2 zusammenzieht. Diese Verbindung ergibt eine deutlich höhere Festigkeit und dynamische Belastbarkeit als die herkömmlichen Lösungen.

Das Zugseil 2 ist vorzugsweise als Geflecht ausgebildet, um eine geringere Dehnbarkeit und eine reibungsärmere Oberfläche zu erreichen. Der Durchmesser des Zugseils 2 beträgt etwa 0,2 mm bis 0,25 mm.

Das hantelförmige Element 1 besteht aus einer NiTi-Legierung. Der erste Endabschnitt 7 und der zweite Endabschnitt 6 des hantelförmigen Elements 1 können beispielsweise mittels Plasmastrahlschmelzen aus einem Drahtabschnitt erzeugt werden. Hierbei werden die Enden des Drahts mittels eines Plasmastrahl(-lichtbogens) inert aufgeschmolzen. Durch die Oberflächenspannung formt sich die lokale Schmelze in dem ersten Endabschnitt 7 und dem zweiten Endabschnitt 6 jeweils zu einem Kügelchen. Das Verfahren ist extrem schnell, reproduzierbar und ermöglicht einen hohen Durchsatz.

Die in Figur 1 gezeigte Verbindung zwischen der Elektrode 9 und dem Zugseil 2 ist an dem distalen Ende des Katheters 14, der in Figur 2 dargestellt ist, angeordnet. Ein erfindungsgemäßer Katheter 14 weist ferner in dieser Reihenfolge in proximaler Richtung einen Zwischenabschnitt 15, einen Katheterkörper 16, einen Steuergriff 18 sowie einen Anschluss 19 für Energie-, Signal-, und/oder Medienzuleitungen auf.

### Bezugszeichenliste:

- 1: hantelförmiges Element
- 2: Zugseil
- 3: Kopfelektrode
- 4: Trompetenknoten
- 5: Palstek
- 6: zweiter Endabschnitt des hantelförmigen Elements 1
- 7: erster Endabschnitt des hantelförmigen Elements 1
- 8: Lot
- 9: Sacklochausnehmung
- 10: Nut
- 11: Mittelabschnitt des hantelförmigen Elements 1
- 14: Katheter
- 15: Zwischenabschnitt
- 16: Katheterkörper
- 18: Steuergriff
- 19: Anschluss für Energie-, Signal-, und/oder Medienzuleitungen

## Patentansprüche

1. Katheter mit einem Steuerungsseilanker (3), vorzugsweise einer Elektrode, wobei der Steuerungsseilanker (3) vorzugsweise am distalen Ende des Katheters (14) angeordnet ist, und mit einem Steuerungsseil (2), **dadurch gekennzeichnet, dass** zwischen dem Steuerungsseilanker (3) und dem Steuerungsseil (2) ein im Wesentlichen hantelförmiges Element (1) angeordnet ist, welches den Steuerungsseilanker (3) mit dem Steuerungsseil (2) verbindet.

2. Katheter nach Anspruch 1, **dadurch gekennzeichnet, dass** das hantelförmige Element (1) einen ersten Endabschnitt (7) und einen zweiten Endabschnitt (6) sowie einen dazwischen liegenden Mittelabschnitt (11) aufweist, wobei vorzugsweise der erste Endabschnitt (7) mit dem Steuerungsseilanker (3) verbunden und an dem zweiten Endabschnitt (6) das Steuerungsseil (2) angeschlagen ist.

3. Katheter nach einem der vorhergehenden Ansprüche, **dadurch gekennzeichnet, dass** das Steuerungsseil (2) ein Polymer enthält.

4. Katheter nach einem der vorhergehenden Ansprüche, **dadurch gekennzeichnet, dass** der Steuerungsseilanker (3) und/oder das hantelförmige Element (1) ein metallisches Material enthalten, wobei das hantelförmige Element (1) vorzugsweise nicht ferromagnetisch ausgebildet ist.

5. Katheter nach einem der vorhergehenden Ansprüche, **dadurch gekennzeichnet, dass** der erste Endabschnitt (7) des hantelförmigen Elements (1) im Wesentlichen formschlüssig mit der Elektrode (3) verbunden ist.

6. Katheter nach einem der vorhergehenden Ansprüche, **dadurch gekennzeichnet, dass** der erste Endabschnitt (7) und/oder der zweite Endabschnitt (6) des hantelförmigen Elements (1) als Kugel, Kugelabschnitt, Kegel, Kegelabschnitt oder Zylinder ausgebildet ist/sind.

7. Katheter nach einem der vorhergehenden Ansprüche, **dadurch gekennzeichnet, dass** das Steuerungsseil (2) an seinem distalen Ende eine Schlaufe (4) ausbildet, welche um den zweiten Endabschnitt (6) des hantelförmigen Elements (1) geschlungen ist.

8. Katheter nach einem der vorhergehenden Ansprüche, **dadurch gekennzeichnet, dass** der erste Endabschnitt (7) und/oder der zweite Endabschnitt (6) des hantelförmigen Elements (1) einen maximalen Durchmesser in radialer Richtung des hantelförmigen Elements (1) aufweist, der mindestens das Doppelte des Durchmessers des Mittelabschnitts (11) in radialer Richtung des hantelförmigen Elements (1) beträgt.

9. Katheter nach einem der vorhergehenden Ansprüche, **dadurch gekennzeichnet, dass** der Mittelabschnitt (11) des hantelförmigen Elements einen Durchmesser von weniger als 1 mm und eine Länge von mindestens 3 mm, vorzugsweise eine Länge von mindestens 5 mm, aufweist.

10. Verfahren zur Herstellung eines Katheters mit einem Steuerungsseilanker (3), welcher vorzugsweise am distalen Ende des Katheters angeordnet und vorzugsweise als Elektrode ausgebildet ist, und mit einem Steuerungsseil (2), mit den folgenden Schritten:
a) Herstellen des Steuerungsseilankers (3) und des Steuerungsseils (2),
b) Herstellen eines hantelförmigen Elements (1) mit einem ersten Endabschnitt (7), einem zweiten Endabschnitt(6) und einem dazwischen liegenden Mittelabschnitt (11),
c) Verbinden des Steuerungsseilankers (3) mit dem ersten Endabschnitt (7) des hantelförmigen Elements (1), vorzugsweise über eine im Wesentlichen formschlüssige Verbindung und
d) Verbinden des Steuerungsseils (2) mit dem zweiten Endabschnitt (6) des hantelförmigen Elements (1), vorzugsweise durch Anschlagen des Steuerungsseils (2) an dem zweiten Endabschnitt (6).

11. Verfahren nach Anspruch 10, **dadurch gekennzeichnet, dass** das Steuerungsseil (2) mittels einer mehrfach angeschlungenen Schlaufe (4) an dem zweiten Endabschnitt (6) des hantelförmigen Elements (1) befestigt wird.

12. Verfahren nach einem der Ansprüche 10 bis 11, **dadurch gekennzeichnet, dass** das hantelförmige Element (1) aus einem Draht, vorzugsweise aus einem Draht einer NiTi-Legierung, gefertigt wird, wobei der erste Endabschnitt (7) und/oder der zweite Endabschnitt (6) vorzugsweise mittels Plasmastrahlschweißen erzeugt werden.
